(19) Europäisches Patentamt
European Patent Office
Office européen des brevets

(11) Publication number: 0 069 448
A1

# (12) EUROPEAN PATENT APPLICATION

(21) Application number: 82302282.7

(22) Date of filing: 05.05.82

(51) Int. Cl.3: C 07 D 249/08
C 07 D 233/56, A 61 K 31/41
A 61 K 31/415

(30) Priority: 19.05.81 GB 8115294

(43) Date of publication of application:
12.01.83 Bulletin 83/2

(84) Designated Contracting States:
BE CH DE FR GB IT LI LU NL SE

(71) Applicant: IMPERIAL CHEMICAL INDUSTRIES PLC
Imperial Chemical House Millbank
London SW1P 3JF(GB)

(72) Inventor: Gravestock, Michael Barry
10 Dawlish Avenue Cheadle Hulme
Cheadle Cheshire(GB)

(74) Representative: Atkinson, John David et al,
Imperial Chemical Industries PLC Legal Department :
Patents Thames House North Millbank
London SW1P 4QG(GB)

## (54) Fungicidal amide derivatives.

(57) 2,2-Bis(substituted phenyl)-3-(triazolyl or imidazolyl)propionamides which possess useful activity against mammalian and plant fungi, together with processes for their manufacture and compositions containing them.



Croydon Printing Company Ltd.

TITLE: FUNGICIDAL AMIDE DERIVATIVES

This invention relates to novel triazole and imidazole compounds which are useful as fungicides, to processes for their manufacture, to fungicidal compositions containing them, and to a method of using them for combatting fungal infections of plants. The fungicidal compositions may be in a form suitable for oral or topical administration to human or other animals for the treatment of fungus diseases, especially candidosis and human dermatophyte infections, or they may be in a form suitable for administration to plants or seeds, or to the surrounding environment thereof.

Triazole and imidazole compounds of various types are known to possess fungicidal properties. In particular, European Specification Number 11768 discloses triazole compounds of the formula:-

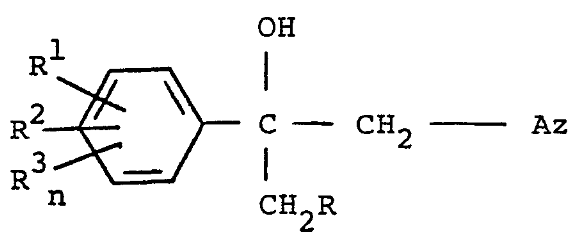

in which Az is a triazolyl radical, R is an optionally substituted phenyl, naphthyl or tetrahydronaphthyl radical, $R^1$ is an optionally substituted phenyl or cycloalkyl radical, $R^2$ is hydrogen or $R^1$ and $R^2$ are in an ortho-relationship and form an optionally substituted polymethylene bridge or together with the phenyl ring form naphthyl, $R^3$ is halogen or an alkyl, alkoxy or halogenoalkyl radical, and n is 0, 1, 2 or 3;

European Specification Number 11769 discloses similar compounds in which the substituent $-CH_2R$ is replaced by an optionally substituted phenyl, naphthyl or tetrahydronaphthyl radical;

and our copending Application Number 8005141 discloses compounds of the formula:-

$$Tr-CH_2-CR^4R^5-OH$$

wherein Tr is a 1,2,4-triazolyl radical, $R^4$ is an alkyl, cycloalkyl or optionally substituted phenyl radical, and $R^5$ is an optionally substituted phenyl or optionally substituted benzyl radical.

According to the present invention there is provided a compound of the formula:-

$$X-CH_2-CR^6R^7-CO-NR^8R^9 \qquad I$$

wherein X is a triazolyl or imidazolyl radical, $R^6$ and $R^7$, which may be the same or different, are each a phenyl radical optionally bearing 1 to 5 substituents selected from halogen atoms and amino, nitro, cyano, phenyl and halogenophenyl radicals, and alkyl, halogenoalkyl, alkoxy, halogenoalkoxy, alkylthio, alkylenedioxy, alkylamino and dialkylamino radicals wherein each alkyl is of 1 to 8 carbon atoms, provided that at least one of $R^6$ and $R^7$ contains at least one substituent, and $R^8$ and $R^9$, which may be the

same or different, are each a hydrogen atom, a cycloalkyl radical of 3 to 7 carbon atoms, an alkyl, alkoxyalkyl, hydroxyalkyl, aminoalkyl, alkylaminoalkyl, dialkylaminoalkyl or carboxyalkyl radical wherein each alkyl is of 1 to 8 carbon atoms, or a phenyl, naphthyl or benzyl radical, optionally substituted as defined above for $R^6$ and $R^7$, or $R^8$ and $R^9$ together with the adjacent nitrogen atom form a 1-pyrrolidinyl or morpholino radical, or a radical of the formula:-

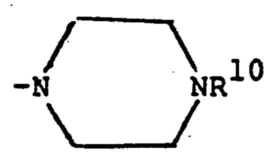

wherein $R^{10}$ is a hydrogen atom, an alkyl radical of 1 to 8 carbon atoms, or alkanoyl radical of 2 to 6 carbon atoms.

When X is a triazolyl radical, it is preferably a 1,2,4-triazolyl radical, and particularly a 1,2,4-triazol-1-yl radical.

The phenyl radicals $R^6$ and $R^7$ preferably contain one or two substituents.

Suitable halogen substituents in $R^6$ and $R^7$, in a halogenophenyl substituent in $R^6$ or $R^7$, and in $R^8$ and $R^9$ when either is a halogen substituted phenyl, naphthyl or benzyl radical, are fluorine, chlorine, bromine and iodine atoms, and of these fluorine and chlorine are preferred.

A preferred value for $R^8$, $R^9$ or $R^{10}$, when any is an alkyl radical, or for an alkyl substituent in $R^6$ or $R^7$, is an alkyl radical of 1-8 carbon atoms, but alkyl radicals of 1-3 carbon atoms are especially preferred. Particular such radicals are methyl, ethyl, propyl, isopropyl, butyl, isobutyl, s-butyl, t-butyl, pentyl, hexyl, heptyl and octyl radicals.

A suitable value for $R^8$ or $R^9$, when either is an alkoxyalkyl, hydroxyalkyl or carboxyalkyl radical, or for a halogenoalkyl, alkoxy, halogenoalkoxy, alkylthio, alkylamino or dialkylamino substituent in $R^6$ or $R^7$, is such a radical wherein each alkyl or alkoxy part is of 1-6, preferably 1-3, carbon atoms. Particular such radicals are therefore, for example, methoxymethyl, 3-methoxypropyl, 6-methoxyhexyl, propoxymethyl, 3-propoxypropyl, 6-propoxyhexyl, hexyloxymethyl, 3-hexyloxypropyl, 6-hexyloxyhexyl, hydroxymethyl, 1-hydroxyethyl, 2-hydroxyethyl, 3-hydroxypropyl, 6-hydroxyhexyl, carboxymethyl, 2-carboxyethyl, 3-carboxypropyl, 6-carboxyhexyl, fluoromethyl, chloromethyl, bromomethyl, iodomethyl, fluorohexyl, chlorohexyl, bromohexyl, iodohexyl, trichloromethyl, trifluoromethyl, 2,2,2-trifluoroethyl, methoxy, ethoxy, propoxy, isopropoxy, butoxy, isobutoxy, s-butoxy, t-butoxy, pentyloxy, hexyloxy, fluoromethoxy, chloromethoxy, bromomethoxy, iodomethoxy, fluorohexyloxy, chlorohexyloxy, bromohexyloxy, iodohexyloxy, 2,2,2-trichloroethoxy, methylthio, ethylthio, propylthio, isopropylthio, butylthio,

pentylthio, hexylthio, methylamino, ethylamino, propylamino, isopropylamino, butylamino, pentylamino, hexylamino, dimethylamino, diethylamino, dipropylamino, dihexylamino, methylethylamino, methylpropylamino and methylhexylamino radicals.

A suitable value for $R^8$ and $R^9$, when either is an aminoalkyl, alkylaminoalkyl or dialkylaminoalkyl radical is such a radical wherein each alkyl is of 1 to 6 preferably 1 to 3 carbon atoms. Particular such radicals are therefore, for example, aminomethyl, 1- or 2-aminoethyl, 1-, 2- or 3-aminopropyl, 1-, 2-, 3-, 4-, 5- or 6-aminohexyl, methylaminomethyl, hexylaminomethyl, methylaminopropyl, hexylaminopropyl, methylaminohexyl, hexylaminohexyl, dimethylaminomethyl, dimethylaminoethyl, dimethylaminopropyl, dimethylaminohexyl and dihexylaminohexyl radicals.

A suitable value for an alkylenedioxy substituent in $R^6$ or $R^7$ is a methylenedioxy radical, or a 1,2-alkylenedioxy radical of 2 to 6 carbon atoms, for example a 1,2-ethylenedioxy, 1,2-propylenedioxy, 1-methyl-1,2-propylenedioxy, 1,2-butylenedioxy, 1-methyl-1,2-butylenedioxy or 1-ethyl-1,2-butylenedioxy radical.

A suitable value for $R^8$ or $R^9$ when either is a cycloalkyl radical is, for example, a cycloalkyl radical of 3 to 7 carbon atoms, that is, a cyclopropyl, cyclobutyl, cyclopentyl, cyclohexyl or cycloheptyl radical.

A suitable value for $R^{10}$ when it is an alkanoyl radical is, for example, such a radical of 2-6 carbon atoms, particularly one of 2-4 carbon atoms, and especially an acetyl, propionyl, butyryl, or valeryl radical.

A preferred group of compounds of the invention comprises those wherein $R^6$ and $R^7$ are each a halogenophenyl radical, preferably a 4-halogenophenyl or a 2,4-dihalogenophenyl radical.

A further preferred group of compounds of the invention comprises those compounds wherein $R^8$ and $R^9$ are each a hydrogen atom, or $R^8$ is a hydrogen atom and $R^9$ is an alkyl radical as defined above.

A further preferred group of compounds of the invention comprises those compounds wherein X is a 1,2,4-triazol-1-yl radical, $R^6$ and $R^7$ are each a halogenophenyl radical, especially a 4-halogenophenyl or 2,4-dihalogenophenyl radical, and $R^8$ and $R^9$ are each a hydrogen atom, or $R^8$ is a hydrogen atom and $R^9$ is an alkyl radical as defined above.

Particular preferred compounds of the invention are 2,2-bis(4-chlorophenyl)-3-(1,2,4-triazol-1-yl)propionamide and 2-(2,4-dichlorophenyl)-2-(4-fluorophenyl)-3-(1,2,4-triazol-1-yl)propionamide.

It will be appreciated that, when $R^6$ and $R^7$ are different, the carbon atom to which they are attached is asymmetrically substituted, and that such compounds may therefore exist in either racemic or optically active form. It is common general

knowledge how such racemic compounds may be resolved to the two optical isomers, and the respective biological properties of the resolved optical isomers determined.

The compounds of the formula I may be manufactured by any of the conventional processes known in the chemical literature for the manufacture of analogous compounds. Thus, the following process is provided as a further feature of this invention, wherein X, $R^6$, $R^7$, $R^8$ and $R^9$ have the meanings stated above:-

(a) the reaction of a compound of the formula:-

$$Z\text{-}CH_2\text{-}CR^6R^7\text{-}CO\text{-}NR^8R^9 \qquad II$$

wherein Z is a leaving group, for example a bromine or chlorine atom or a methanesulphonyl or toluenesulphonyl radical, with a compound of the formula XH, either in the presence of an acid-binding agent or in the form of one of its alkali metal salts in a convenient solvent;

(b) for those compounds wherein $R^8$ and $R^9$ are each a hydrogen atom, the hydrolysis with an acid or a base of the corresponding nitrile of the formula:-

$$X\text{-}CH_2\text{-}CR^6R^7\text{-}CN \qquad III$$

(c) the reaction of a compound of the formula $X\text{-}CH_2\text{-}CR^6R^7\text{-}COOH$, or of an acid chloride or bromide, active ester or salt thereof, with an amine of the formula $R^8R^9NH$.

In process (a), the reaction may conveniently be carried out using the sodium salt of the compound XH (prepared by adding an anhydrou strong sodium base such as sodium hydride or sodium methoxide to XH) in a convenient organic solvent, for example methanol, ethanol, dimethylformamide or acetonitrile, at a temperature in the range 20-100°C.

The acid used in hydrolysis process (b) may conveniently be sulphuric acid or hydrochloric acid, and the reaction is typically carried out by heating the reaction mixture to an elevated temperature in the range 40-100°C. for 2-6 hours.

The compounds of the formula II used as the starting materials in process (a) may be obtained, for example, as follows:-

a nitrile $R^6CH_2.CN$ (IV) or $R^7CH_2.CN$ (V) is brominated to $R^6CHBr.CN$ or $R^7CHBr.CN$ (VI), which is reacted with a compound $R^7H$ or $R^6H$ respectively in the presence of a Lewis acid, to give a compound $R^6R^7CH.CN$ (VII). Compound VII may, if desired be hydrolysed to the corresponding amide $R^6R^7CH.CONH_2$ (VIII) which may in turn be alkylated or arylated if desired to a substituted amide $R^6R^7CH.CONR^8R^9$ (IX). Alternatively, substituted amides IX may be obtained by reacting the corresponding acid, $R^6R^7CH.COOH$ or a lower alkyl ester thereof, with the appropriate amine, $NHR^8R^9$. An amide VIII or substituted amide IX is then reacted with formaldehyde to give a hydroxymethyl compound, $HOCH_2-CR^6R^7-CO-NR^8R^9$ (X), which is in turn halogenated, mesylated or tosylated to give the required starting material II.

The starting material of the formula III used in process (b) is obtained in similar manner from a nitrile VII, by reaction with formaldehyde to give a hydroxymethyl compound, $HOCH_2.CR^6R^7.CN$, which is halogenated, mesylated or tosylated and then reacted with a compound XH to give the starting material III.

As indicated above, the compounds of the invention possess antifungal properties which are useful in the treatment of candidosis and human dermatophyte infections.

This antifungal activity against Candida albicans, a causative fungus of candidosis, and Trichophyton mentagrophytes, var. quinkeanum,a causative fungus of ringworm, was demonstrated as follows:-

Female mice of around 30g. weight are injected sub-cutaneously on a Friday with 0.5mg. of oestradiol benzoate. The following Monday (day 0) they are clipped on the back and then dosed orally with test compounds. They are then inoculated with Candida albicans in the vagina and Trichophyton mentagrophytes var. quinkeanum on the back, and then given a second dose of the same compound. Dosing is repeated once daily on days 1-4. On day 7 skin lesions are scored visually and vaginal samples taken for culture on agar. Groups of 5 mice are used and compounds are dosed initially at a level of 250mg./kg. The dose is then reduced sequentially until a minimum effective dose (MED) is found. The MED for 2,2-bis(4-chlorophenyl)-3-(1,2,4-triazol-1-yl)propionamide in this test was 5mg. per kg.

Thus, according to a further feature of the invention there is provided a pharmaceutical or veterinary fungicidal compositi ı which comprises a compound of the formula I together with a pharmaceutically or veterinarily acceptable diluent or carrier.

The composition of the invention may be in a conventional pharmaceutical form suitable for oral administration, for example a tablet, a capsule, an emulsion or an aqueous or oily solution or suspension, or suitable for topical application, for example a cream, ointment or gel. The composition may contain conventional pharmaceutical excipients, and may be manufactured by conventional pharmaceutical techniques.

Preferred pharmaceutical or veterinary compositions of the invention are compositions suitable for oral administration, and particularly tablets and capsules.

The compounds of the invention also possess antifungal properties which are useful in combatting plant fungal diseases, and the compounds are effective particularly against the diseases:-

*Piricularia oryzae* on rice;
*Puccinia recondita*, *Puccinia striiformis* and other rusts on wheat, *Puccinia hordei*, *Puccinia striiformis* and other rusts on barley; and rusts on other hosts e.g. coffee, apples, vegetables and ornamental plants;
*Plasmopara viticola* on vines;
*Erysiphe graminis* (powdery mildew) on barley and wheat; and other powdery mildews on various hosts such as

*Sphaerotheca fuliginea* on cucurbits (e.g. cucumber), *Podosphaera leucotricha* on apples and *Uncinula necator* on vines;

*Helminthosporium* spp. and *Rhynchosporium* spp. on cereals;

*Cercospora arachidicola* on peanuts and other *Cercospora* species on for example sugar beet, bananas and soya beans;

*Botrytis cinerea* (grey mould) on tomatoes, strawberries, vines and other hosts;

*Phytophthora infestans* (late blight) on tomatoes; and *Venturia inaequalis* (scab) on apples.

Some of the compounds have also shown a broad range of activities against fungi *in vitro*. They have activity against various post-harvest diseases on fruit (e.g. *Penicillium digatatum* and *italicum* on oranges and *Gloeosporium musarum* on bananas). Further some of the compounds are active as seed dressing against: *Fusarium* spp., *Septoria* spp., *Tilletia* spp. (i.e. bunt, a seed borne disease of wheat), *Ustilago* spp., *Helminthosporium* spp. on cereals, *Rhizoctonia solani* on cotton and *Corticium sasakii* on rice.

The compounds can move acropetally in the plant tissue. Moreover, the compounds can be volatile enough to be active in the vapour phase against fungi on the plant.

The compounds may be used as such for fungicidal purposes but are more conveniently formulated into compositions for such usage.

The invention thus provides also a plant fungicidal composition comprising a compound of general formula (I) and a carrier or diluent.

The invention also provides a method of combatting fungal diseases in a plant, which method comprising applying to the plant, to seed of the plant or to the locus of the plant or seed a compound of the formula I.

The compound can be applied in a number of ways, for example it can be formulated or unformulated, directly to the foliage of a plant, to seeds or to other medium in which plants are growing or are to be planted, or it can be sprayed on, dusted on or applied as a cream or paste formulation, or it can be applied as a vapour. Application can be to any part of the plant, bush or tree, for example to the foliage, stems, branches or roots, or to soil surrounding the roots, or to the seed before it is planted.

The term "plant" as used herein includes seedlings, bushes and trees. Furthermore, the fungicidal method of the invention includes preventative, protectant, prophylactic and eradicant treatment.

The compounds are preferably used for agricultural and horticultural purposes in the form of a composition. The type of composition used in any instance will depend upon the particular purpose envisaged.

The compositions may be in the form of dusting powders or granules comprising the active ingredient and a solid diluent or carrier, for example fillers such as kaolin, bentonite, kieselguhr, dolomite, calcium carbonate, talc, powdered magnesia, Fuller's earth, gypsum, Hewitt's earth, diatomaceous earth and China clay. Such granules can be preformed granules suitable for application to the soil without further treatment. These granules can be made either by impregnating pellets of filler with the active ingredient or by pelleting a mixture of the active ingredient and powdered filler. Compositions for dressing seed, for example, may comprise an agent (for example a mineral oil) for assisting the adhesion of the composition to the seed; alternatively the active ingredient can be formulated for seed dressing purposes using an organic solvent (for example N-methylpyrrolidone or dimethylformamide).

The compositions may also be in the form of dispersible powder, granules or grains comprising a wetting agent to facilitate the dispersion in liquids of the powder or grains which may contain also fillers and suspending agents.

The aqueous dispersions or emulsions may be prepared by dissolving the active ingredient(s) in an organic solvent optionally containing wetting, dispersing or emulsifying agent(s) and then adding the mixture to water which may also contain wetting, dispersing or emulsifying agent(s). Suitable organic solvents are ethylene dichloride, isopropyl

alcohol, propylene glycol, diacetone alcohol, toluene, kerosene, methylnaphthalene, the xylenes, trichloroethylene, furfuryl alcohol, tetrahydrofurfuryl alcohol, and glycol esters (e.g. 2-ethoxyethanol and 2-butoxyethanol).

The compositions to be used as sprays may also be in the form of aerosols wherein the formulation is held in a container under pressure in the presence of a propellant, e.g. fluorotrichloromethane or dichlorodifluoromethane.

The compounds can be mixed in the dry state with a pyrotechnic mixture to form a composition suitable for generating in enclosed spaces a smoke containing the compounds.

Alternatively, the compounds may be used in a microencapsulated form.

By including suitable additives, for example additives for improving the distribution, adhesive power and resistance to rain on treated surfaces, the different compositions can be better adapted for various utilities.

The compounds can be used as mixtures with fertilisers (e.g. nitrogen-, potassium- or phosphorus-containing fertilisers). Compositions comprising only granules of fertiliser incorporating, for example coated with, the compound, are preferred. Such granules suitably contain up to 25% by weight of the compound. The invention therefore also provides a fertiliser composition comprising the compound of general formula (I).

The compositions may also be in the form of liquid preparation for use as dips or sprays which are generally aqueous dispersions or emulsions containing the active ingredient in the presence of one or more surfactants e.g. wetting agent(s), dispersing agent(s), emulsifying agent(s) or suspending agent(s). These agents can be cationic, anionic or non-ionic agents. Suitable cationic agents are quaternary ammonium compounds, for example cetyltrimethylammonium bromide.

Suitable anionic agents are soaps, salts of aliphatic monoesters of sulphuric acid (for example sodium lauryl sulphate), and salts of sulphonated aromatic compounds (for example sodium dodecylbenzenesulphonate, sodium, calcium or ammonium lignosulphonate, butyl-naphthalene sulphonate, and a mixture of sodium diisopropyl- and triisopropylnaphthalene sulphonates).

Suitable non-ionic agents are the condensation products of ethylene oxide with fatty alcohols such as oleyl or cetyl alcohols, or with alkyl phenols such as octyl- or nonyl-phenol and octylcresol. Other non-ionic agents are the partial esters derived from long chain fatty acids and hexitol anhydrides, the condensation products of the said partial esters with ethylene oxide, and the lecithins. Suitable suspending agents are hydrophilic colloids (for example polyvinylpyrrolidone and sodium carboxymethylcellulose), and the vegetable gums (for example gum acacia and gum tragacanth).

The compositions for use as aqueous dispersions or emulsions are generally supplied in the form of a concentrate containing a high proportion of the active ingredient(s), the concentrate to be diluted with water before use. The concentrates often should be able to withstand storage for prolonged periods and after such storage be capable of dilution with water in order to form aqueous preparations which remain homogeneous for a sufficient time to enable them to be applied by conventional spray equipment. The concentrates may conveniently contain up to 95%, suitably 10-85%, for example 25-60%, by weight of the active ingredient(s). These concentrates suitably contain organic acids (e.g. alkaryl or aryl sulphonic acids such as xylenesulphonic acid or dodecylbenzenesulphonic acid) since the presence of such acids can increase the solubility of the active ingredient(s) in the polar solvents often used in the concentrates. The concentrates suitably contain also a high proportion of surfactants so that sufficiently stable emulsions in water can be obtained. After dilution to form aqueous preparations, such preparations may contain varying amounts of the active ingredient(s) depending upon the intended purpose, but an aqueous preparation containing 0.0005% or 0.01% to 10% by weight of active ingredient(s) may be used.

The plant fungicidal compositions of this invention can comprise also other compound(s) having biological activity, e.g. compounds having similar or complement ary fungicidal activity or compounds having plant growth regulating, herbicidal or insecticidal activity.

The other fungicidal compound can be for example one which is capable of combatting ear diseases of cereals (e.g. wheat) such as *Septoria*, *Gibberella* and *Helminthosporium* spp., seed- and soil-borne diseases, downy and powdery mildews on grapes, and powdery mildew and scab on apples etc. These mixtures of fungicides can have a broader spectrum of activity than the compound of general formula (I) alone; further the other fungicide can have a synergistic effect on the fungicidal activity of the compound of general formula (I). Examples of the other fungicidal compound are imizalil, benomyl, carbendazim, thiophanate-methyl, captafol, captan, sulphur, triforine, dodemorph, tridemorph, pyrazophos, furalaxyl, ethirimol, dimethirimol, bupirimate, chlorothalonil, vinclozolin, procymidone, iprodione, metalaxyl, forsetyl-aluminium, carboxyin, oxycarboxin, fenarimol, nuarimol, fenfuram, methoxyfuroxan, nitrotal-isopropyl, triadimefon, thiabendazole, etrifiazole, triadimenol, biloxazol, dithianon, binapacryl, quinomethionate, guazitine, dodine, fentin acetate, fentin hydroxide, dinocap, folpet, dichlofluanid, ditalimphos, kitazin, cycloheximide, dichlorobutrazol, a dithiocarbamate, a copper compound, a mercury compound, DPX 3217, RH 2161, Chevron RE 20615, CGA 64250, CGA 64251 and RO 14-3169.

The compounds of general formula (I) can be mixed with soil, peat or other rooting media for the protection of plants against seed-borne, soil-borne or foliar fungal diseases.

Suitable insecticides are pirimor, croneton, dimethoate, metasystox and formothion.

Examples of suitable plant growth regulating compounds are the gibberellins (e.g. $GA_3$, $GA_4$ or $GA_7$), the auxins (e.g. indoleacetic acid, indolebutyric acid, naphthoxyacetic acid or naphthylacetic acid), the cytokinins (e.g. kinetin, diphenylurea, benzimidazole, benzyladenine or BAP), phenoxyacetic acid (e.g. 2,4-D or MCPA), substituted benzoic acids (e.g. TIBA), morphactins (e.g. chlorfluorecol), maleic hydrazide, glyphosate, glyphosine, long chain fatty alcohols and acids (e.g. Off Shoot O or Off Shoot T), dikegulac, Sustar, Embark, substituted quaternary ammonium and phosphonium compounds (e.g. CCC or Phosfon-D), Ethrel, carbetamide, Racuza, Alar, asulam, abscissic acid, isopyrimol, RH531, hydroxybenzonitriles (e.g. bromoxynil), Avenge, Suffix or Lontrel.

The invention is illustrated but not limited, by the following Examples; in which "parts" are by weight:-

Example 1

A mixture of 2,2-bis(4-chlorophenyl)-3-(1,2,4-triazol-1-yl)propionitrile (1.4g.) and concentrated sulphuric acid (5ml.) was heated at 60°C. for 4 hours, and then poured into cold water (25ml.) and extracted with ethyl acetate. The ethyl acetate phase was separated, washed twice with brine, dried over sodium sulphate and filtered, and the filtrate was evaporated to dryness under

reduced pressure. The résidual solid was crystallised from a mixture of ethyl acetate and petroleum ether (b.p. 60-80°C.) to give 2,2-bis(4-chlorophenyl)-3-(1,2,4-triazol-1-yl)propionamide, m.p. 186°C.

Example 2

A mixture of 5, 10, 25, 50, 100 or 250 parts of 2,2-bis(4-chlorophenyl)-3-(1,2,4-triazol-1-yl)propionamide with 70 parts of calcium carbonate and 200 parts of a 10% maize starch past is dried and then passed through a 16 mesh screen. 5 parts of magnesium stearate are added and the granules are compressed to give a range of tablets suitable for oral administration for therapeutic purposes.

This active ingredient may be replaced by a therapeutically equivalent amount of any other triazole derivatives as hereinbefore defined.

Example 3

A mixture of 2, 5, 10, 25, 50 or 100 parts of 2,2-bis(4-chlorophenyl)-3-(1,2,4-triazol-1-yl)-propionamide, 500 parts of lactose and 100 parts of maize starch is treated with sufficient 10% maize starch paste to give a granular mass. Each mixture is passed through a 16-mesh screen, dried, mixed with 8 parts of magnesium stearate and compressed into tablets, thus giving a range of tablets suitable for oral administration for therapeutic purposes.

The active ingredient may be replaced by a therapeutically equivalent amount of any other triazole derivatives as hereinbefore defined.

Example 4

A mixture of 10 parts of 2,2-bis(4-chlorophenyl)-3-(1,2,4-triazol-1-yl)propionamide and 190 parts of wheat germ oil is filled into soft gelatin capsules, to give capsules suitable for oral administration for therapeutic purposes.

The active ingredientr may be replaced by a therapeutically equivalent amount of any other triazole derivative as hereinbefore defined.

Example 5

A solution of 10 parts of 2,2-bis(4-chlorophenyl)-3-(1,2,4-triazol-1-yl)propionamide in 83 parts of water, 250 parts of glycerol and 125 parts of ethyl alcohol is mixed with a solution of 300 parts of sucrose in 150 parts of water. A suitable flavouring agent and colouring matter are then added to produce a syrup suitable for oral administration for therapeutic purposes.

The active ingredient may be replaced by a therapeutically equivalent amount of any other triazole derivative as hereinbefore defined.

Example 6

A mixture of 3 parts of gum acacia and 1.5 parts of gum tragacanth is added to a mixture of 1 part of 2,2-bis(4-chlorophenyl)-3-(1,2,4-triazol-1-yl)propionamide and 33.7 parts of liquid paraffin. To the thoroughly triturated mixture is added slowly with stirring a solution of 0.1 part of cetyl alcohol-polyoxyethylene condensate, 40 parts of sucrose, 0.03 part of propyl p-hydroxybenzoate,

0.3 part of methyl p-hydroxybenzoate, a suitable flavouring agent and 0.002 part of edible dyestuff in 110 parts of water. The mixture is then homogenized in conventional manner known in the art to produce an emulsion suitable for oral administration for therapeutic purposes.

The active ingredient may be replaced by a therapeutically equivalent amount of any other triazole derivative as hereinbefore defined.

Example 7

A mixture of 0.5 part of finely divided 2,2-bis(4-chlorophenyl)-3-(1,2,4-triazol-1-yl)-propionamide in 3 parts of propylene glycol and 2 parts of ethylene glycol monoethyl ether was added to a stirred mixture of 4 parts of lanolin and 90.5 parts of molten soft white paraffin. The resulting mixture was allowed to cool to room temperature with rapid stirring, to give a uniform ointment containing 0.5% by weight of active ingredient suitable for topical administration for therapeutic purposes.

The active ingredient may be replaced by another triazole derivative as hereinbefore defined to give similar ointments.

Example 8

A solution was prepared of 1 part of 2,2-bis(4-chlorophenyl)-3-(1,2,4-triazol-1-yl)-propionamide in 20 parts of ethanol and 27 parts of diethylene glycol monoethyl ester, then 50 parts of purified water was added, followed by 2 parts of a carboxypolymethylene gelling agent ("Carbapol 940" - trade mark) to give a finely dispersed gel suitable for topical administration for therapeutic purposes.

The active ingredient may be replaced by any other triazole derivative as hereinbefore described.

In the following Examples 9 to 18 the proportion of the ingredients given are by weight. The active ingredient in each Example is 2,2-bis-(4-chlorophenyl)-3-(1,2,4-triazol-1-yl)propionamide. Substances represented therein by various Trade Marks and Trade Names are characterised as follows:-
1 mole) with ethylene oxide

| | | |
|---|---|---|
| LUBROL L | : | a condensate of nonyl phenol (1 mole) with ethylene oxide (13 moles) |
| AROMASOL H | : | a solvent mixure of alkyl-benzenes |
| DISPERSOL T & AC | : | a mixture of sodium sulphate and a condensate of formaldehyde with sodium naphthalene sulphonate |
| LUBROL APN5 | : | a condensate of nonyl phenol (1 mole) with naphthalene oxide (5.5 moles) |

| | | |
|---|---|---|
| CELLOFAS B600 | : | a sodium carboxymethyl cellulose thickener |
| LISSAPOL NX | : | a condensate of nonyl phenol (1 mole) with ethylene oxide (8 moles) |
| AEROSOL OT/B | : | dioctyl sodium sulphosuccinate |
| PERMINAL BX | : | a sodium alkyl naphthalene |

Example 9

An emulsifiable concentate was made up by mixing the ingredients, and stirring the mixture until all the constituents were dissolved.

| | |
|---|---|
| Active ingredient | 10% |
| Ethylene dichloride | 40% |
| Calcium dodecylbenzenesulphonate | 5% |
| "Lubrol" L | 10% |
| "Aromasol" H | 35% |

Example 10

A composition in the form of grains readily dispersible in a liquid, e.g. water, was prepared by grinding together the first three ingredients in the presence of added water and then mixing in the sodium acetate. The resultant mixture was dried and passed through a British Standard mesh sieve, size 44-100, to obtain the desired size of grains.

| | |
|---|---|
| Active ingredient | 50% |
| "Dispersol" T | 25% |
| "Lubrol" APN5 | 1.5% |
| Sodium acetate | 23.5% |

Example 11

The ingredients were all ground together to produce a powder formulation readily dispersible in liquids.

| | |
|---|---|
| Active ingredient | 45% |
| "Dispersol" T | 5% |
| "Lissapol" NX | 0.5% |
| "Cellofas" B600 | 2% |
| Sodium acetate | 47.5% |

Example 12

The active ingredient was dissolved in a solvent and the resultant liquid was sprayed on to the granules of China clay. The solvent was then allowed to evaporate to produce a granular composition.

| | |
|---|---|
| Active ingredient | 5% |
| China clay granules | 95% |

Example 13

A composition suitable for use as a seed dressing was prepared by mixing the three ingredients.

| | |
|---|---|
| Active ingredient | 50% |
| Mineral oil | 2% |
| China clay | 48% |

Example 14

A dust powder was prepared by mixing the active ingredient with talc.

| | |
|---|---|
| Active ingredient | 5% |
| Talc | 95% |

Example 15

A col formulation was prepared by ball-milling the constituents set out below and then forming an aqueous suspension of the ground mixture with water.

| | |
|---|---|
| Active ingredient | 40% |
| "Dispersol" T | 10% |
| "Lubrol" APN5 | 1% |
| Water | to 100% |

Example 16

A dispersible powder formulation was made by mixing together the ingredients set out below and then grinding the mixture until all were thoroughly mixed.

| | |
|---|---|
| Active ingredient | 25% |
| "Aerosol" OT/B | 2% |
| "Dispersol" A.C. | 5% |
| China clay | 28% |
| Silica | 40% |

Example 17

This Example illustrates the preparation of a dispersible powder formulation. The ingredients were mixed and the mixture then ground in a comminution mill.

| | |
|---|---|
| Active ingredient | 25% |
| "Perminal" BX | 1% |
| "Dispersol" T | 5% |
| Polyvinylpyrrolidone | 10% |
| Silica | 25% |
| China clay | 34% |

Example 18

The ingredients set out below were formulated into a dispersible powder by mixing then grinding the ingredients.

| | |
|---|---|
| Active ingredient | 25% |
| "Aerosol" OT/B | 2% |
| "Dispersol" A | 5% |
| China clay | 68% |

Example 19

The compound of Example 1 was tested against a variety of foliar fungal diseases of plants. The technique employed was as follows:-

The plants were grown in John Innes Potting Compost (No. 1 or 2) in 4 cm diameter minipots. A

layer of fine sand was placed at the bottom of the pots containing the dicotyledonous plants to facilitate uptake of test compound by the roots. The test compound was formulated either by bead milling with aqueous Dispersol T or as a solution of acetone or acetone/ethanol which was diluted to the required concentration immediately before use. For the foliage diseases, suspensions (100 ppm active ingredient) were sprayed on to the soil. Exceptions to this were the tests on Botrytis cinerea, Plasmopara viticola and Venturia inaequalis. The sprays were applied to maximum retention and the root drenches to a final concentration equivalent to approximately 40 ppm a.i./dry soil. Tween 20, to give a final concentration of 0.05%, was added when the sprays were applied to cereals.

For most of the tests the compound was applied to the soil (roots) and the foliage (by spraying) one or two days before the plant was inoculated with the diseases. An exception was the test on Erysiphe graminis in which the plants were inoculated 24 hours before treatment. After inoculation, the plants were put into an appropriate environment to allow infection to take place and

The disease control was recorded by the following grading:-

4 = no disease
3 = trace - 5% of disease on untreated plants
2 = 6-25% of disease on untreated plants
1 = 26-59% of disease on untreated plants
0 = 60-100% of disease on untreated plants

The results are shown in the following Table.

| PUCCINIA RECONDITA (WHEAT) | ERYSIPHE GRAMINIS (BARLEY) | PIRICULARIA ORYZAE (RICE) | BOTRYTIS CINEREA (TOMATO) | CERCOSPORA ARACHIDICOLA (PEANUT) | VENTURIA INAEQUALIS (APPLE) |
|---|---|---|---|---|---|
| 1 | 4 | - | - | 2 | 3 |

Example 20

The process described in Example 1 was repeated, using the appropriate nitrile starting materials, and the following compounds were obtained:-

(a) 2-(2-chlorophenyl)-2-(4-chlorophenyl)-3-(1,2,4-triazol-1-yl)propionamide, as a foam; characteristic peaks in the n.m.r. spectrum in deuteriated dimethylsulphoxide as follows:-

$\delta$ 5.38 (quartet, 2H, $-C\underline{H}_2-N-$)
7.10 (multiplet, 2H, $-N\underline{H}_2$)
7.35 (multiplet, 8H, aromatic)
7.62 (singlet, 1H, $-N-C\underline{H}=N-$)
7.90 (singlet, 1H, $-N-C\underline{H}=N-$)

(b) 2-(2,4-dichlorophenyl)-2-(4-fluorophenyl)-3-(1,2,4-triazol-1-yl)propionamide, crystallised from ethyl acetate/petroleum ether (b.p. 60-80°C.), m.p. 166-167°C.

Example 21

2-(2,4-Dichlorophenyl)-2-(4-fluorophenyl)-3-(1,2,4-triazol-1-yl)propionamide (1.5g.) was dissolved in tetrahydrofuran (25ml.), and sodium hydride

(50% dispersion in oil, 200mg.) was added in portions. The mixture was stirred for 30 minutes, then n-propyl bromide was added dropwise, and the mixture was stirred for 12 hours, then cooled. The solvent was evaporated under reduced pressure, the residue was dissolved in ethyl acetate, the solution was washed with brine twice, then dried, and the solvent was evaporated under reduced pressure. The residual oil was run down a column of silica gel in diethyl ether, and the eluate was evaporated to give 2-(2,4-dichlorophenyl)-2-(4-fluorophenyl)-N-n-propyl-3-(1,2,4-triazol-1-yl)propionamide as a colourless oil. Characteristic peaks in the n.m.r. spectrum in deuteriochloroform were seen as follows:-

$\delta$ 0.79 (triplet, 3H, methyl)
1.31 (multiplet, 2H, $-C\underline{H}_2.CH_3-$)
3.20 (quartet, 2H, $-CO.NH.C\underline{H}_2-$)
5.40 (singlet, 2H, $-C\underline{H}_2.N<$)
6.15 (triplet, 1H, $-CONH\underline{H}-$)
7.20 (multiplet, 7H, aromatic)
7.66 (singlet, 1H, -N.CH=N-)
8.13 (singlet, 1H, -N-CH=N-)

The process described above was repeated using n-octyl bromide in place of n-propyl bromide, to give 2-(2,4-dichlorophenyl)-2-(4-fluorophenyl)-N-n-octyl-3-(1,2,4-triazol-1-yl)propionamide, which was reacted with a solution of hydrogen chloride in ether to afford the hydrochloride salt, m.p. 156-158°C.

WHAT WE CLAIMS IS:-

1. A compound of the formula:-

$$X\text{-}CH_2\text{-}CR^6R^7\text{-}CO\text{-}NR^8R^9 \qquad I$$

wherein X is a triazolyl or imidazolyl radical, $R^6$ and $R^7$, which may be the same or different, are each a phenyl radical optionally bearing 1 to 5 substituents selected from halogen atoms and amino, nitro, cyano, phenyl and halogenophenyl radicals, and alkyl, halogenoalkyl, alkoxy, halogenoalkoxy, alkylthio, alkylenedioxy, alkylamino and dialkylamino radicals wherein each alkyl is of 1 to 8 carbon atoms, provided that at least one of $R^6$ and $R^7$ contains at least one substituent, and $R^8$ and $R^9$, which may be the same or different, are each a hydrogen atom, a cycloalkyl radical of 3 to 7 carbon atoms, an alkyl, alkoxyalkyl, hydroxyalkyl, aminoalkyl, alkylaminoalkyl, dialkylaminoalkyl or carboxyalkyl radical wherein each alkyl is of 1 to 8 carbon atoms, or a phenyl, naphthyl or benzyl radical, optionally substituted as defined above for $R^6$ and $R^7$, or $R^8$ and $R^9$ together with the adjacent nitrogen atom form a 1-pyrrolidinyl or morpholino radical, or a radical of the formula:-

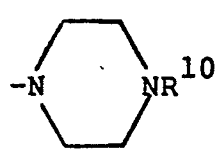

wherein $R^{10}$ is a hydrogen atom, alkyl radical of 1 to 8 carbon atoms or alkanoyl radical of 2 to 7 carbon atoms.

2. A compound as claimed in claim 1 wherein X is a 1,2,4-triazolyl or imidazolyl radical, $R^6$ and $R^7$ which may be the same or different, are each a phenyl radical optionally bearing 1 to 5 substituents selected from fluorine, chlorine, bromine and iodine atoms and amino, nitro, cyano, phenyl, fluorophenyl, chlorophenyl, bromophenyl, iodophenyl, methyl, ethyl, propyl, isopropyl, butyl, isobutyl, s-butyl, t-butyl, pentyl, hexyl, heptyl, octyl, fluoromethyl, chloromethyl, bromomethyl, iodomethyl, fluorohexyl, chlorohexyl, bromohexyl, iodohexyl, trichloromethyl, trifluoromethyl, 2,2,2-trifluoroethyl, methoxy, ethoxy, propoxy, isopropoxy, butoxy, isobutoxy, s-butoxy, t-butoxy, pentyloxy, hexyloxy, fluoromethoxy, chloromethoxy, bromomethoxy, iodomethoxy, chlorohexyloxy, fluorohexyloxy, bromohexyloxy, iodohexyloxy, 2,2,2-trichloroethoxy, methylthio, ethylthio, propylthio, isopropylthio, butylthio, pentylthio, hexylthio, 1,2-ethylenedioxy, 1,2-propylenedioxy, 1-methyl-1,2-propylenedioxy, 1,2-butylenedioxy, 1-methyl-1,2-butylenedioxy or 1-ethyl-1,2-butylenedioxy, methylamino, ethylamino, propylamino, isopropylamino, butylamino, pentylamino, hexylamino, dimethylamino, diethylamino, dipropylamino, dihexylamino, methylethylamino, methylpropylamino and methylhexylamino radicals, and $R^8$ and $R^9$, which may be the same or different are each a hydrogen atom, a cyclopropyl, cyclobutyl, cyclopentyl, cyclohexyl or cycloheptyl radical, a methoxymethyl, 3-methoxypropyl, 6-methoxyhexyl, propoxymethyl, 3-propoxypropyl, 6-propoxyhexyl, hexyloxymethyl, 3-hexyloxypropyl, 6-hexyloxyhexyl, hydroxymethyl, 1-hydroxyethyl, 2-hydroxyethyl, 3-hydroxypropyl, 6-hydroxyhexyl, aminomethyl, 1- or 2-aminoethyl, 1-, 2- or 3-aminopropyl, 1-, 2-, 3-, 4-, 5- or 6-aminohexyl, methylaminomethyl, hexylaminomethyl, methylaminopropyl, hexylaminopropyl, methylaminohexyl, hexylaminohexyl, dimethylaminomethyl, dimethylaminoethyl, dimethylaminopropyl, dimethylaminohexyl, dihexylaminohexyl, carboxymethyl, 2-carboxyethyl, 3-carboxypropyl, 6-carboxyhexyl or phenyl radical or a substituted phenyl radical as defined above for $R^5$ and $R^6$, or $R^8$ and $R^9$ together with the adjacent nitrogen atom form a 1-pyrrolidinyl or morpholino radical, or a radical of the formula:-

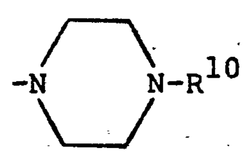

wherein $R^{10}$ is a hydrogen atom or a methyl, ethyl, propyl, butyl, pentyl, hexyl, heptyl, octyl, acetyl, propionyl, butyryl or valeryl radical.

3. A compound as claimed in claim 1 or 2 wherein $R^6$ and $R^7$ are each a halogenophenyl radical.

4. A compound as claimed in any one of claims 1,2 and 3 wherein $R^8$ and $R^9$ are each a hydrogen atom, or $R^8$ is a hydrogen atom and $R^9$ is an alkyl radical.

5. A compound as claimed in claim 1 wherein X is a 1,2,4-triazol-1-yl radical, $R^6$ and $R^7$ are each a 4-halogenophenyl or 2,4-dihalogenophenyl radical and $R^8$ and $R^9$ are each a hydrogen atom or $R^8$ is a hydrogen atom and $R^9$ is an alkyl radical.

6. The compounds 2,2-bis(4-chlorophenyl)-3-(1,2,4-triazol-1-yl)propionamide and 2-(2,4-dichlorophenyl)-2-(4-fluorophenyl)-3-(1,2,4-triazol-1-yl)-propionamide.

7. A process for the manufacture of a compound as claimed in claim 1 which comprises:-

(a) the reaction of a compound of the formula:-

$$Z-CH_2-CR^6R^7-CO-NR^8R^9 \qquad II$$

wherein Z is a leaving group, for example a bromine or chlorine atom or a methanesulphonyl or toluenesulphonyl radical, with a compound of the formula XH, either in the presence of an acid-binding agent or in the form of one of its alkali metal salts in a convenient solvent;

(b) for those compounds wherein $R^8$ and $R^9$ are each a hydrogen atom, the hydrolysis with an acid or a base of the corresponding nitrile of the formula:-

$$X-CH_2-CR^6R^7-CN \qquad III$$

(c) the reaction of a compound of the formula $X-CH_2-CR^6R^7-COOH$, or of an acid chloride or bromide, active ester or salt thereof, with an amine of the formula $R^8R^9NH$.

wherein X, $R^6$, $R^7$, $R^8$ and $R^9$ have the meanings stated in claim 1.

8. A pharmaceutical or veterinary fungicidal composition which comprises a compound of the formula I together with a pharmaceutically or veterinarily acceptable diluent or carrier.

9. A plant fungicidal composition comprising a compound of general formula I as claimed in claim 1 and a carrier or diluent.

10. A method of combatting fungal diseases in a plant, which method comprises applying to the plant, to seed of the plant or to the locus of the plant or seed a compound of the formula I, as claimed in claim 1.

JDA/LMS : PH.31850 : 31 Mar 82

JOHN DA... ..KINSON

AUTHORISED REPRESENTATIVE

General Authorisation No. 97

European Patent Office

**0069448**

Application number

# EUROPEAN SEARCH REPORT

EP 82 30 2282

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| A | EP-A-0 017 850 (BAYER) | | C 07 D 249/08<br>C 07 D 233/56<br>A 61 K 31/41<br>A 61 K 31/415 |
| A | EP-A-0 018 510 (CIBA-GEIGY) | | |
| A | EP-A-0 013 873 (BASF) | | |
| D,A | EP-A-0 011 768 (BAYER) | | |
| D,A | EP-A-0 011 769 (BAYER) | | |
| P | EP-A-0 046 633 (I.C.I.) | | |
| | ----- | | **TECHNICAL FIELDS SEARCHED (Int. Cl. ³)**<br>C 07 D 249/00<br>C 07 D 233/00<br>A 61 K 31/00 |

The present search report has been drawn up for all claims

| Place of search | Date of completion of the search | Examiner |
|---|---|---|
| THE HAGUE | 26-08-1982 | DE BUYSER I.A.F. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons

& : member of the same patent family, corresponding document